(19) Europäisches Patentamt
European Patent Office
Office européen des brevets

(11) EP 4 446 428 A1

(12) # EUROPEAN PATENT APPLICATION
published in accordance with Art. 153(4) EPC

(43) Date of publication:
**16.10.2024 Bulletin 2024/42**

(21) Application number: **22904120.7**

(22) Date of filing: **01.12.2022**

(51) International Patent Classification (IPC):
***C12Q 1/6827*** *(2018.01)* ***C12N 15/11*** *(2006.01)*

(52) Cooperative Patent Classification (CPC):
**C12Q 1/6827;** C12N 15/11

(86) International application number:
**PCT/JP2022/044333**

(87) International publication number:
**WO 2023/106189 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.12.2021 JP 2021201160**

(71) Applicant: **Nitto Boseki Co., Ltd.**
**Fukushima-shi,**
**Fukushima 960-8161 (JP)**

(72) Inventors:
- **FUJIMURA Nahohiro**
  **Kawasaki-shi, Kanagawa 210-0821 (JP)**
- **TACHIBANA Ami**
  **Kawasaki-shi, Kanagawa 210-0821 (JP)**
- **UCHIKI Tomoaki**
  **Kawasaki-shi, Kanagawa 210-0821 (JP)**
- **TAKEUCHI Minoru**
  **Koriyama-shi, Fukushima 963-8061 (JP)**
- **TERUUCHI Yoko**
  **Koriyama-shi, Fukushima 963-8061 (JP)**
- **WATANABE Koji**
  **Koriyama-shi, Fukushima 963-8061 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **AGENT FOR INCREASING MELTING TEMPERATURE (TM VALUE) OF NUCLEIC ACID**

(57) Provided is a new agent for increasing the melting temperature (Tm value) of a nucleic acid. The present invention uses a specific (di)allylamine-based compound that meets a specific relationship between the molecular weight of the (di)allylamine-based compound and a numerical value calculated from a specific parameter related to the chemical structure of the (di)allylamine-based compound.

EP 4 446 428 A1

## Description

TECHNICAL FIELD

The present invention relates to an agent for raising a melting temperature (Tm value) of a nucleic acid (hereinafter, may also be referred to as an agent for raising a Tm value). More specifically, the present invention relates to an agent for raising a melting temperature (Tm value) of a nucleic acid (particularly, a double-stranded nucleic acid), the agent comprising a (di)allylamine-based compound with a specific structure.

BACKGROUND ART

Nucleic acids are subjected to molecular biological analysis in many fields such as medical fields, food fields, agricultural or fishery fields, and environmental analysis, or are used as an active ingredient of therapeutic drugs, and one of the problems associated therewith is to stabilize nucleic acids in a sample. Conventionally, as an attempt to stabilize a nucleic acid, for example, a mixed solution of ethanol and acetone, ammonium sulfate, tetradecyltrimethylammonium oxalate, and the like have been proposed to stabilize a nucleic acid(for example, Patent Literature 1).

Further, in order to identify specific bacteria and viruses or to determine disease susceptibility, drug response, and the like in a human subject, mutations in a genomic nucleic acid are detected, and as one of means for the detection, a melting temperature (Tm value) of the nucleic acid is utilized.

The melting temperature (Tm value) means a temperature at which 50% of a double-stranded nucleic acid is dissociated into single strands, and the melting temperature (Tm value) is lower in a double strand having a mismatch and thus not a fully complementary relation between double-stranded nucleic acids as compared with a double strand of which the strands are fully complementary. Accordingly, mutations in a sample nucleic acid with respect to a standard nucleic acid can be detected by utilizing this phenomenon, or a target sequence can be detected through a difference in nucleic acid amplification efficiency caused by this phenomenon. However, a change in melting temperature (Tm value) caused by one base mismatch is considered about 1 to 3°C. Therefore, in order to detect a very small number of mutations in a genomic nucleic acid by means of using a melting temperature (Tm value), it is necessary to improve the sensitivity.

In addition, a low annealing temperature in a nucleic acid amplification reaction may cause non-specific amplification, which requires examining the GC content of a primer and designing a primer with an annealing temperature of about 55°C (NON-Patent Literature 1). If a primer can be designed with reduced dependency on GC content, more flexible primer design will be allowed.

In this regard, a method of analyzing a melting curve to detect a very small number of mutations has been developed (Patent Literature 2). However, this method requires a highly accurate thermostatic apparatus capable of strictly controlling temperatures, and a more convenient method is desired for practical use. It also does not address the problem of primer design dependent on GC content.

In the meantime, although it is not a method of detecting mutations using a melting temperature (Tm value), there has been proposed a method of detecting a mismatch with a standard gene by means of using a cationic macromolecular polymer that accelerates the rate of replacing a short-chain nucleic acid molecule having a mismatch with the standard gene, with a short-chain nucleic acid molecule having a full match, and this method is considered capable of detecting even one base mismatch (Patent Literatures 3 to 5). This cationic macromolecular polymer is a polymer obtained by graft-polymerizing a cationic polymer such as polylysine or polyarginine as a main chain and dextran or polyethylene glycol as a side chain, and as specific examples thereof, α-PLL-g-Dex, ε-PLL-g-Dex, and PAA-g-Dex are disclosed. Patent Literature 3 shows a difference between the Tm values of a nucleic acid molecule having a single base mutation in the presence and absence of this cationic macromolecular polymer. Unfortunately, the Tm rise value due to the addition of this cationic macromolecular polymer is 15°C or less for a 20-mer DNA, which does not sufficiently increase the detection sensitivity in a method of detecting mutations using a Tm value, nor does it sufficiently increase the annealing temperature in a nucleic acid amplification reaction.

CITATION LIST

PATENT LITERATURE

PATENT LITERATURE 1: JP-A-2015-212273
PATENT LITERATURE 2: JP-A-2005-58107
PATENT LITERATURE 3: WO 03/018841 A1

PATENT LITERATURE 4: JP-A-2001-78769
PATENT LITERATURE 5: JP-A-2008-278779

NON-PATENT LITERATURE

NON-PATENT LITERATURE 1: Dieffenbach et al., "General concepts for PCR primer design", PCR Methods Appl., 1993, 3(3), S30-37

SUMMARY OF INVENTION

TECHNICAL PROBLEM

An object of the present invention is to provide a novel agent for raising the melting temperature (Tm value) of a nucleic acid, which can address the problems in the prior art as described above. Another object of the present invention is to provide a pharmaceutical composition comprising the agent for raising the Tm value of a nucleic acid together with a nucleic acid.

SOLUTION TO PROBLEM

As a result of intensive studies, the inventors of the present invention have found that adding a specific (di)allylamine-based compound in which a numerical value calculated from a specific parameter related to its chemical structure and its molecular weight M satisfy a specific relationship to a nucleic acid solution can effectively raise the melting temperature (Tm value) of a nucleic acid, thereby completing the present invention.

That is, as one embodiment, the present invention provides the following agent for raising the melting temperature (Tm value) of a nucleic acid.

[1] An agent for raising a melting temperature (Tm value) of a nucleic acid, comprising:

a (di)allylamine-based compound, wherein the (di)allylamine-based compound comprises at least one type of (di)allylamine-based constituent unit selected from the group consisting of
a constituent unit (A) having a structure represented by General Formula (I-a), General Formula (I-b), or General Formula (I-c), or a structure of an acid addition salt thereof:

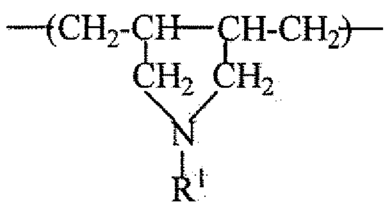

(I-a)

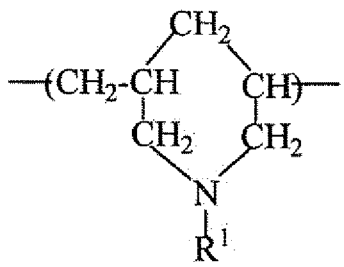

(I-b)

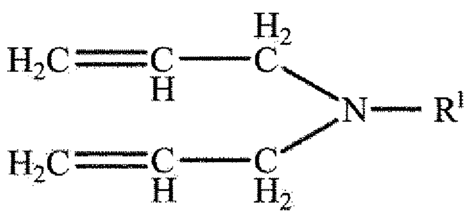

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

a constituent unit (B) having a structure represented by General Formula (I-d), General Formula (I-e), or General Formula (I-f):

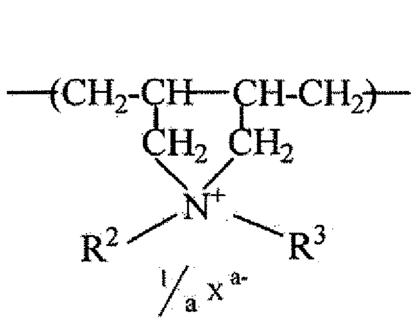

(I-d)

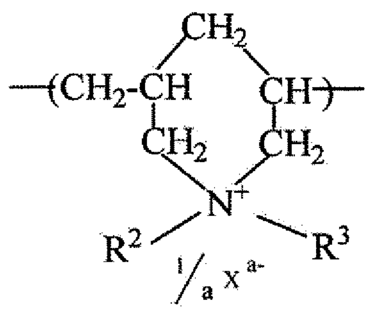

(I-e)

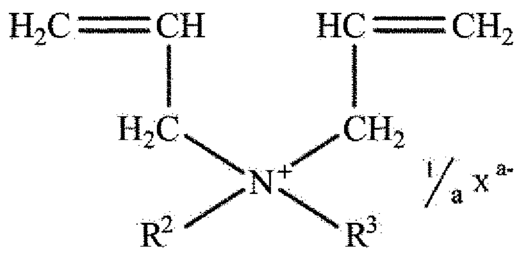

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and a constituent unit (C) having a structure represented by General Formula (I-g) or General Formula (1-h), or a structure of an acid addition salt thereof:

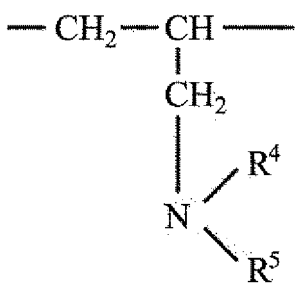

(I-g)

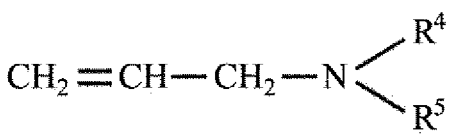

(I-h)

wherein $R^4$ and $R^5$ each independently represent a hydrogen atom, an alkyl group having 1 to 12 carbon atoms

that may have a hydroxyl group, an aralkyl group having 7 to 10 carbon atoms, or a cycloalkyl group having 5 to 6 carbon atoms; and wherein

the (di)allylamine-based compound is a compound in which a chemical structure parameter P calculated by the following Formula (1) and a molecular weight M (specified by a weight average molecular weight when the (di)allylamine-based compound is a (di)allylamine-based (co)polymer) are in a relationship satisfying the following Formula (2):

$$P = \frac{\sum_{i=1}^{n} m_i\left(\sum_{j=1}^{4} j \times a_{ij} + o_i + 2 \times s_i\right)}{\sum_{i=1}^{n} m_i} \quad \cdots (1)$$

wherein n represents the number of constituent units constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i.

$$0.84 \leqq P^3/M^{1/4} \leqq 12.67 \quad \cdots (2)$$

The invention also provides other embodiments as described in [2] to [19] below.

[2] The agent for use in raising a melting temperature (Tm value) of a nucleic acid according to [1], comprising a (di)allylamine-based compound in which the P and the M are in a relationship satisfying the following Formula (3):

$$2.37 \leqq P^3/M^{1/4} \leqq 6.92 \quad \cdots (3)$$

[3] The agent for use in raising a melting temperature (Tm value) of a nucleic acid according to [1], comprising a (di)allylamine-based compound in which the P and the M are in a relationship satisfying the following Formula (4):

$$2.60 \leqq P^3/M^{1/4} \leqq 3.00 \quad \cdots (4)$$

[4] The agent for use in raising a melting temperature (Tm value) of a nucleic acid according to any one of [1] to [3], wherein the (di)allylamine-based compound is a (di)allylamine-based (co)polymer.

[5] The agent for use in raising a melting temperature (Tm value) of a nucleic acid according to any one of [1] to [4], wherein the nucleic acid is DNA.

[6] A method for stabilizing a nucleic acid, comprising:
adding the agent for raising a melting temperature (Tm value) of a nucleic acid according to any one of [1] to [5] to an aqueous solution containing a nucleic acid.

[7] An aqueous solution comprising the agent for raising a melting temperature (Tm value) of a nucleic acid according to any one of [1] to [5] together with a nucleic acid.

[8] Use of a (di)allylamine-based compound for producing an agent for raising a melting temperature (Tm value) of a nucleic acid, comprising:

a (di)allylamine-based compound, wherein the (di)allylamine-based compound comprises at least one type of (di)allylamine-based constituent unit selected from the group consisting of

a constituent unit (A) having a structure represented by General Formula (I-a), General Formula (I-b), or General Formula (I-c), or a structure of an acid addition salt thereof:

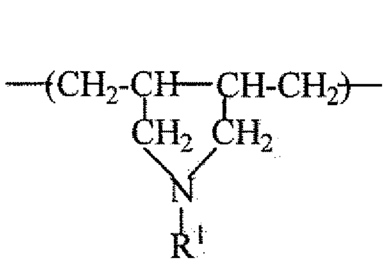 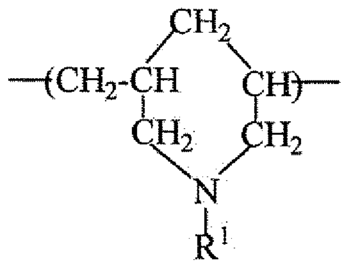

(I-a) (I-b)

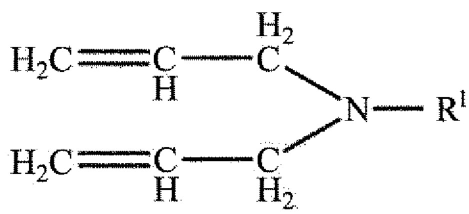

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

a constituent unit (B) having a structure represented by General Formula (I-d), General Formula (I-e), or General Formula (I-f):

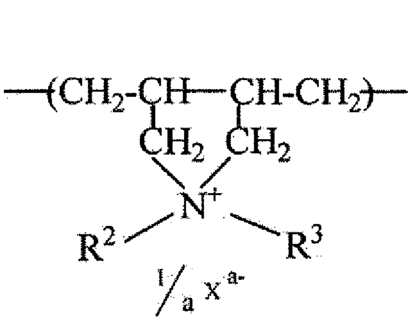 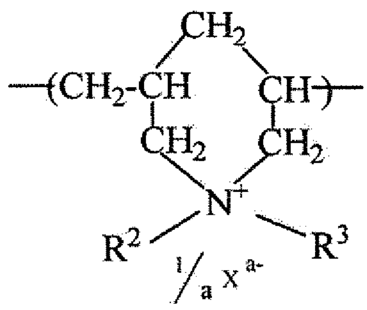

(I-d) (I-e)

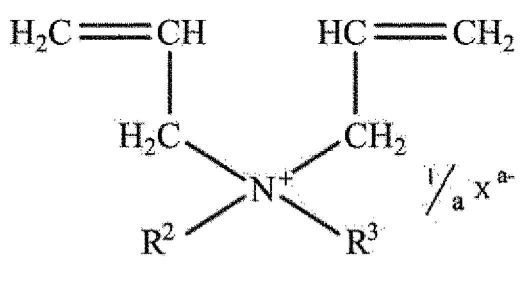

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and

a constituent unit (C) having a structure represented by General Formula (I-g) or General Formula (1-h), or a structure

of an acid addition salt thereof:

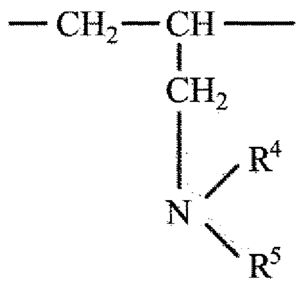

(I-g)

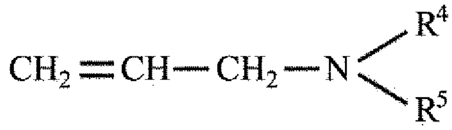

(I-h)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms; and wherein

the (di)allylamine-based compound is a compound in which a chemical structure parameter P calculated by the following Formula (1) and a molecular weight M (specified by a weight average molecular weight when the (di)allylamine-based compound is a (di)allylamine-based (co)polymer) are in a relationship satisfying the following Formula (2):

$$P = \frac{\sum_{i=1}^{n} m_i \left( \sum_{j=1}^{4} j \times a_{ij} + o_i + 2 \times s_i \right)}{\sum_{i=1}^{n} m_i} \quad \cdots \quad (1)$$

wherein n represents the number of constituent units constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i.

$$0.84 \leqq P^3/M^{1/4} \leqq 12.67 \quad \cdots \quad (2)$$

[9] The use according to [8], wherein the (di)allylamine-based compound is a compound in which the P and the M are in a relationship satisfying the following Formula (3):

$$2.37 \leqq P^3/M^{1/4} \leqq 6.92 \quad \cdots \quad (3)$$

[10] The use according to [8], wherein the (di)allylamine-based compound is a compound in which the P and the M are in a relationship satisfying the following Formula (4).

$$2.60 \leqq P^3/M^{1/4} \leqq 3.00 \quad \cdots \quad (4)$$

[11] The use according to any one of [8] to [10], wherein the (di)allylamine-based compound is a (di)allylamine-based (co)polymer.

[12] The use according to any one of [8] to [11], wherein the nucleic acid is DNA.

[13] A method for stabilizing a nucleic acid, comprising adding the (di)allylamine-based compound according to any one of [1] to [4] to an aqueous solution containing a nucleic acid.

[14] An aqueous solution comprising the (di)allylamine-based compound according to any one of [1] to [4] together with a nucleic acid.

[15] A method for raising a melting temperature (Tm value) of a nucleic acid, comprising

mixing the nucleic acid with a (di)allylamine-based compound in a solution, wherein the (di)allylamine-based compound comprises: at least one type of (di)allylamine-based constituent unit selected from the group consisting of a constituent unit (A) having a structure represented by General Formula (I-a), General Formula (I-b), or General Formula (I-c), or a structure of an acid addition salt thereof:

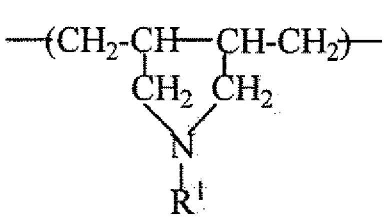

(I-a)

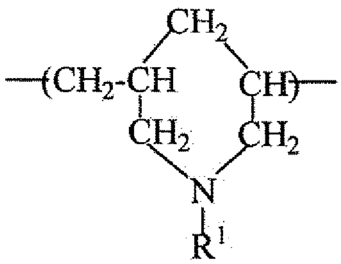

(I-b)

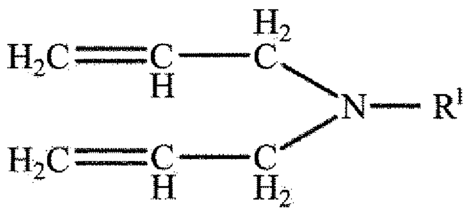

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

a constituent unit (B) having a structure represented by General Formula (I-d), General Formula (I-e), or General Formula (I-f):

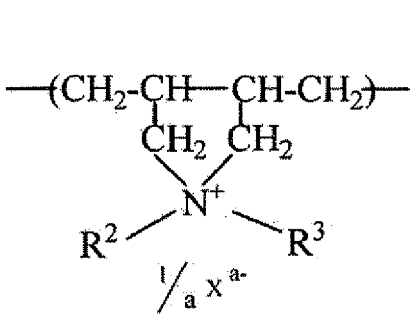

(I-d)

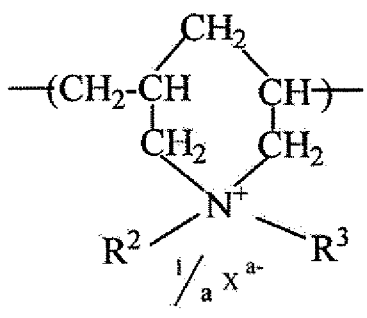

(I-e)

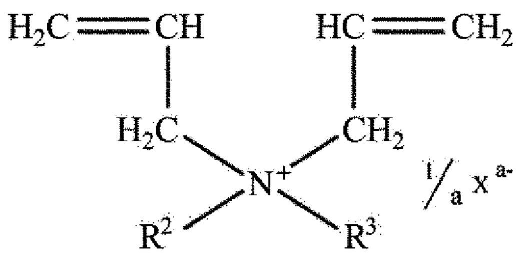

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and a constituent unit (C) having a structure represented by General Formula (I-g) or General Formula (1-h), or a structure of an acid addition salt thereof:

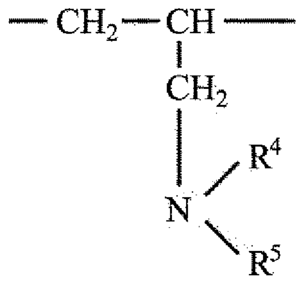

(I-g)

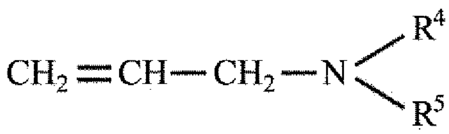

(I-h)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms; and wherein the (di)allylamine-based compound is a compound in which a chemical structure parameter P calculated by the following Formula (1) and a molecular weight M (specified by a weight average molecular weight when the (di)allylamine-based compound is a (di)allylamine-based (co)polymer) are in a relationship satisfying the following Formula (2):

$$P = \frac{\sum_{i=1}^{n} m_i\left(\sum_{j=1}^{4} j \times a_{ij} + o_i + 2 \times s_i\right)}{\sum_{i=1}^{n} m_i} \quad \cdots \quad (1)$$

wherein n represents the number of constituent units constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i.

$$0.84 \leq P^3/M^{1/4} \leq 12.67 \quad \cdots (2)$$

[16] The method according to [15], wherein the (di)allylamine-based compound is a compound in which the P and the M are in a relationship satisfying the following Formula (3):

$$2.37 \leq P^3/M^{1/4} \leq 6.92 \quad \cdots (3)$$

[17] The method according to [15], wherein the (di)allylamine-based compound is a compound in which the P and the M are in a relationship satisfying the following Formula (4):

$$2.60 \leq P^3/M^{1/4} \leq 3.00 \quad \cdots (4)$$

[18] The method according to any one of [15] to [17], wherein the (di)allylamine-based compound is a (di)allylamine-based (co)polymer.

[19] The method according to any one of [15] to [18], wherein the nucleic acid is DNA.

The agent for raising a Tm value of a nucleic acid of the present invention comprises the above-mentioned (di)allylamine-based compound with a specific structure and remarkably raises the Tm value of a nucleic acid. This agent is expected to allow detection of mutations in a genomic nucleic acid with higher sensitivity. In addition, this agent is expected to stabilize a nucleic acid when added to compositions containing the nucleic acid.

Here, the "melting temperature (Tm value)" means a temperature at which 50% of a double-stranded nucleic acid are dissociated into single strands, and as used herein this means a Tm value obtained by measuring fluorescence intensity at each 0.3°C rise from 40°C (10°C if the Tm value cannot be measured in case of raising the temperature from 40°C or if the Tm value is less than 50°C) to 95°C in a real-time PCR system using an intercalator to create a double-strand melting curve, and determining the Tm value from this double-strand melting curve by a differentiation method.

DESCRIPTION OF EMBODIMENTS

An agent for raising the Tm value of a nucleic acid of the present invention comprises a (di)allylamine-based compound having a (di)allylamine-based constituent unit with a specific structure, in which a parameter calculated from the chemical structure thereof and the molecular weight M thereof satisfy a specific relationship (hereinafter, the compound is also referred to as a "specific compound").

An agent for raising a Tm value of a nucleic acid of the present invention may consist of the above-described specific compound, or may comprise other components in addition to the specific compound.

Specific Compound

The above-described specific compound has a (di)allylamine-based constituent unit with a specific structure as described above, and may be a (co)polymer having a plurality of (di)allylamine-based constituent units (hereinafter, also referred to as a "specific (co)polymer") or may be a monomer having a single (di)allylamine-based constituent unit (hereinafter, also referred to as a "specific monomer"). From the viewpoints of storage stability, an effect of raising a Tm value, and the like, the specific compound is preferably a diallylamine (co)polymer having a plurality of (di)allylamine-based constituent units (i.e., a specific (co)polymer).

Specific (Co)Polymer

A specific (co)polymer, which is a preferred embodiment of a specific compound, is a (di)allylamine-based (co)polymer which comprises:

at least one type of (di)allylamine-based constituent unit ($\alpha$) selected from the group consisting of

a constituent unit (A1) having a structure represented by General Formula (I-a) or General Formula (I-b), or a structure of an acid addition salt thereof:

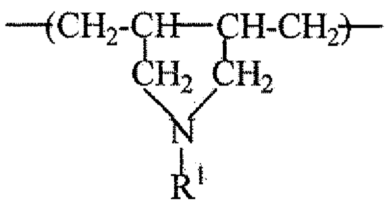

(I-a)

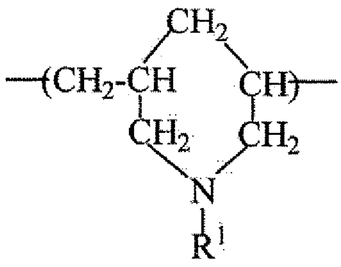

(I-b)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

a constituent unit (B1) having a structure represented by General Formula (I-d) or General Formula (I-e):

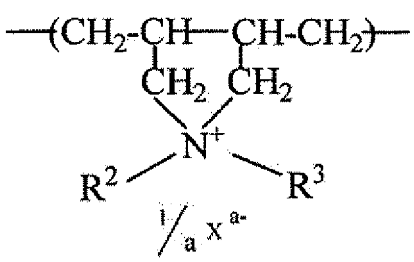

(I-d)

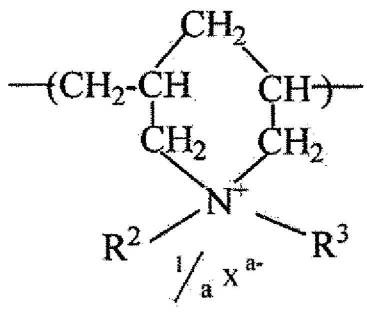

(I-e)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and

a constituent unit (C1) having a structure represented by General Formula (1-g), or a structure of an acid addition salt thereof:

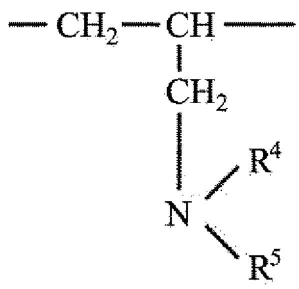

(I-g)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms; and

in which a chemical structure parameter P calculated by the following Formula (1) and a weight average molecular weight M obtained by GPC measurement are in a relationship satisfying the following Formula (2):

$$P = \frac{\sum_{i=1}^{n} m_i \left( \sum_{j=1}^{4} j \times a_{ij} + o_i + 2 \times s_i \right)}{\sum_{i=1}^{n} m_i} \quad \cdots \quad (1)$$

wherein n represents the number of constituent units constituting the (co)polymer, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i. Here, the "j-class amines" are counted based on structures apparently corresponding to "j-class amines". In addition, the "carbonyl group" is counted based on structure apparently corresponding to a "carbonyl group". For example, in a case where acrylamide is a constituent unit, the nitrogen atom in the amide group is counted as a primary amine, and the oxygen atom in the amide group is counted as one carbonyl group.

$$0.84 \leqq P^3/M^{1/4} \leqq 12.67 \quad \cdots (2)$$

Here, when the structures of constituent units constituting a copolymer are known, the molar ratio of each constituent unit constituting the copolymer can be specified by reprecipitating the copolymer with an organic solvent such as isopropyl alcohol or acetone, and analyzing the reprecipitates in appropriate modes according to the known structures of the constituent units constituting the copolymer with a Perkin Elmer 2400II CHNS/O fully automated elemental analyzer or an apparatus having equivalent performance.

In addition, the measurement can be performed by using helium gas as a carrier gas, weighing a solid sample into a tin capsule, dropping the sample into a combustion tube to burn the sample at a combustion temperature of 1800°C or higher in pure oxygen gas, detecting each component of interest by a frontal chromatography system with a separation column and a thermal conductivity detector, and quantifying the content of each element using a calibration factor. Further, in a case where the structures of cationic constituent units and anionic constituent units of a copolymer are unknown, the structures of the constituent units constituting the copolymer are specified by a known method with 1H-NMR or 13C-NMR prior to the above-mentioned measurement by the elemental analyzer. Further, the calculation may be performed with the amount of each monomer supplied in production (copolymerization) of a copolymer, and the amount of each monomer remaining without being incorporated into the copolymer. In this regard, the proportion (molar ratio) of the constituent unit derived from each monomer in the copolymer is considered almost corresponding to the composition (molar ratio) provided for respective constituent units, and the ratio of a monomer may be treated as the proportion (molar ratio) of a corresponding constituent unit for convenience herein.

(Di)Allylamine-Based Constituent Unit (α)

As described above, a specific (co)polymer has a (di)allylamine-based constituent unit with a specific structure (hereinafter, also referred to as "(di)allylamine-based constituent unit (α)").

A specific (co)polymer may consist of a (di)allylamine-based constituent unit (α) or may further comprise a constituent unit other than a (di)allylamine-based constituent unit (α). Examples of constituent units other than a (di)allylamine-based constituent unit (α) include various cationic constituent units other than a (di)allylamine-based constituent unit (α), various nonionic constituent units, and various anionic constituent units.

The proportion of the above-described (di)allylamine-based constituent unit having a specific structure to the whole constituent units of a specific (co)polymer is not particularly limited, but is usually 40 mol% or more, preferably 50 to 100 mol%, more preferably 70 to 100 mol%, and particularly preferably 80 to 100 mol%.

Further, when a nonionic constituent unit is contained as a constituent unit other than the (di)allylamine-based constituent unit (α), the proportion of the (di)allylamine-based constituent unit having a specific structure as the total of the (di)allylamine-based constituent unit (α) and the nonionic constituent unit to the whole constituent units of a specific (co)polymer is usually 50 mol% or more, preferably 70 to 100 mol%, more preferably 80 to 100 mol%, and particularly preferably 90 to 100 mol%.

The (di)allylamine-based constituent unit (α) constituting a specific (co)polymer is more specifically at least one cationic constituent unit selected from the group consisting of the following constituent unit (A1), constituent unit (B1), and constituent unit (C1).

A specific (co)polymer may comprise only one type of (di)allylamine-based constituent unit (α), or may comprise two or more types of (di)allylamine-based constituent units (α).In case of containing two or more types of (di)allylamine-based constituent units (α), they may be a combination of two or more types of constituent units that are all classified into a constituent unit (A1), a combination of two or more types of constituent units that are all classified into a constituent unit (B1), or a combination of two or more types of constituent units that are all classified into a constituent unit (C1), or may be a combination of constituent units that are classified into different ones among the constituent units (A1) to (C1).

All of constituent units (A1) to (C1), which are capable of constituting a specific (co)polymer, have an amino group, but from the viewpoint of achieving a high level of melting temperature (Tm value)- raising effect, constituent units (A1) to (C1) preferably have a secondary, tertiary or quaternary amino group, more preferably have a tertiary or quaternary amino group, and particularly preferably have a quaternary amino group. From this viewpoint, a specific

(co)polymer preferably has at least one type of constitute unit corresponding toa constituent unit (B1) as a (di)allylamine-based constituent unit ($\alpha$).

Constituent Unit (A1)

A constituent unit (A1) is a constituent unit with a structure represented by the following General Formula (I-a) or General Formula (I-b), or a structure of an acid addition salt thereof.

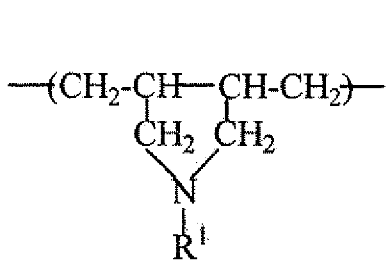

(I-a)

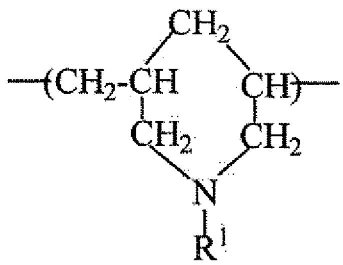

(I-b)

In the formula, $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms. $R^1$ is preferably an alkyl group having 1 to 10 carbon atoms that may have a hydroxyl group, a cycloalkyl group having 5 to 10 carbon atoms, or an aralkyl group having 7 to 10 carbon atoms, more preferably an alkyl group having 1 to 4 carbon atoms, still more preferably a methyl group or an ethyl group, and particularly preferably a methyl group.

From the viewpoint of increasing the value of the chemical structure parameter P, or the like, it is preferable that at least part of constituent units (A1) have a tertiary amino group. The proportion of the constituent units having a tertiary amino group to the whole constituent units (A1) is preferably 50 mol% or more, more preferably 70 to 100 mol%, and still more preferably 90 to 100 mol%.

A constituent unit (A1) may be an inorganic acid salt, an organic acid salt, or the like of the structure represented by the above Structural Formula (I-a) or (I-b), that is, a structure of an acid addition salt thereof.

In a case where a specific (co)polymer has a constituent unit (A1), from the viewpoint of production costs, and the like, an addition salt type of diallylamine monomer is preferably used in producing a specific (co)polymer. A process of removing an addition salt such as HCl from a polymer is complicated and causes an increase in cost, and it is preferable in terms of cost and the like to utilize an addition salt-type constituent unit (A1), which can be produced without requiring such a process.

From the viewpoint of the ease of availability, controllability of reaction, and the like, an inorganic acid salt or organic acid salt of a constituent unit (A1) according to this embodiment is preferably a hydrochloride, a carboxylate, a sulfonate, or an alkyl sulfate, and particularly preferably a hydrochloride.

Constituent Unit (B 1)

A constituent unit (B 1) is a constituent unit with a structure represented by the following General Formula (I-d) or General Formula (I-e).

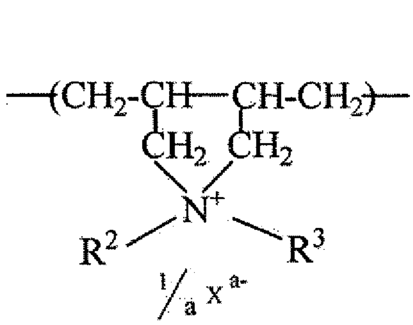

(I-d)

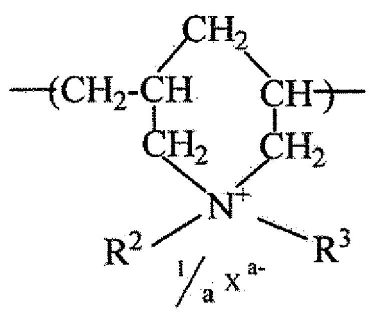

(I-e)

In the formula, $R^2$ and $R^3$ each independently represent a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion.

It is preferable that at least one of $R^2$ and $R^3$ is not a hydrogen atom, and it is more preferable that both are not a hydrogen atom.

$R^2$ and $R^3$ are each independently preferably a methyl group, an ethyl group, or a benzyl group, more preferably a methyl group or an ethyl group, and particularly preferably a methyl group.

From the viewpoint of increasing the value of the chemical structure parameter P, or the like, it is preferable that at least part of constituent units (B 1) have a tertiary or quaternary amino group or ammonium salt. The proportion of the constituent units having a tertiary or quaternary amino group or ammonium salt to the whole constituent units (B 1) is preferably 50 mol% or more, more preferably 70 to 100 mol%, and still more preferably 90 to 100 mol%.

There is no particular limitation on the counter ion $X^{a-}$, but from the viewpoint of the ease of availability, the controllability of reaction, and the like, the counter ion $X^{a-}$ is preferably a chloride ion, a carboxylic acid ion, a sulfonic acid ion, or an alkyl sulfate ion, and particularly preferably a chloride ion, or an ethyl sulfate ion.

From the viewpoint of production costs, and the like, a diallylamine monomer having a counter ion is preferably used in producing a specific (co)polymer. A process of removing a counter ion from a (co)polymer is complicated and causes an increase in cost, and it is preferable in terms of cost and the like to use a (co)polymer having a counter ion-type constituent unit (B 1), which can be produced without requiring such a process.

Constituent Unit (C1)

A constituent unit (C 1) is a constituent unit having a structure represented by the following General Formula (I-g), or a structure of an acid addition salt thereof.

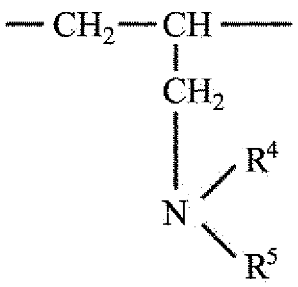

(I-g)

In the formula, $R^4$ and $R^5$ independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms.

It is preferable that at least one of $R^4$ and $R^5$ is not a hydrogen atom, and it is more preferable that both are not a hydrogen atom.

The alkyl group having 1 to 12 carbon atoms and aralkyl group having 7 to 10 carbon atoms, which are preferable as $R^4$ and $R^5$, may be linear or branched. Examples of the groups include a methyl group, an ethyl group, an n-propyl group, an isopropyl group, a n-butyl group, an isobutyl group, a sec-butyl group, a tert-butyl group, a pentyl group, a hexyl group, an octyl group, a decyl group, a dodecyl group, and a benzyl group. Further, examples of the cycloalkyl group having 5 to 6 carbon atoms, which is preferable as $R^4$ and $R^5$, include, but are not limited to, a cyclopentyl group, and a cyclohexyl group.

From the viewpoint of increasing the value of the chemical structure parameter P, and the like, it is preferable that at least part of constituent units (C1) have a tertiary amino group. The proportion of the constituent units having a tertiary amino group to the whole constituent units (C1) is preferably 50 mol% or more, more preferably 70 to 100 mol%, and still more preferably 90 to 100 mol%.

There is no particular limitation on the type of an addition salt in a case where the constituent unit (C1) is an acid addition salt having a structure represented by General Formula (I-e), but from the viewpoint of the ease of availability, controllability of reaction, and the like, the acid addition salt can be, for example, a hydrochloride, a sulfate, a phosphate, a nitrate, a sulfite, a phosphite, a nitrite, a hydrobromide, an acetate, an amide sulfate, a methanesulfonate, a trifluoroacetate, a p-toluenesulfonate, or the like.

Among them, a hydrochloride, a sulfate, a phosphate, and an amide sulfate are preferable, and a hydrochloride,

a sulfate, a phosphate, and an amide sulfate of a structure derived from monoallylamine are particularly preferable.

A specific (co)polymer, which is a preferred aspect of a specific compound, is a (di)allylamine-based (co)polymer in which a chemical structure parameter P calculated by the following Formula (1) and a weight average molecular weight M obtained by GPC measurement are in a relationship satisfying the following Formula (2).

$$P = \frac{\sum_{i=1}^{n} m_i \left( \sum_{j=1}^{4} j \times a_{ij} + o_i + 2 \times s_i \right)}{\sum_{i=1}^{n} m_i} \quad \cdots \quad (1)$$

wherein n represents the number of constituent units constituting a (co)polymer, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i.

$$0.84 \leqq P^3 / M^{1/4} \leqq 12.67 \quad \cdots \quad (2)$$

When the chemical structure parameter P and the weight average molecular weight M of a specific (co)polymer are in the relationship defined by the above Formula (2), an agent for raising the melting temperature (Tm value) of a nucleic acid containing the specific (co)polymer according to this embodiment can effectively increase the Tm value of a hybridized double-stranded nucleic acid.

<u>Chemical Structure Parameter P</u>

The chemical structure parameter P of a specific (co)polymer is a value calculated by the following Formula (1), and is a parameter of combining and then comprehensively representing the degrees to which various groups, including amines, in the constituent units constituting a (co)polymer contribute to the effect of the present embodiment.

$$P = \frac{\sum_{i=1}^{n} m_i \left( \sum_{j=1}^{4} j \times a_{ij} + o_i + 2 \times s_i \right)}{\sum_{i=1}^{n} m_i} \quad \cdots \quad (1)$$

In the formula, n represents the number of constituent units constituting a specific (co)polymer, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i.

Here,

$$\sum_{j=1}^{4} j \times a_{ij}$$

represents the total contribution of an amine or an ammonium salt in the constituent unit i, and a larger contribution is added as the class number j of the amine or ammonium salt is larger. $o_i$ represents the contribution of a carbonyl group in the constituent unit i, and contribution equivalent to that of the same number of primary amines is added in this case. $2 \times s_i$ indicates contribution of a sulfonyl group in the constituent unit i, and contribution equivalent to that of the same number of secondary amines is added in this case.

Contribution of the constituent unit i calculated in this manner:

$$\left( \sum_{j=1}^{4} j \times a_{ij} + o_i + 2 \times s_i \right)$$

is subjected to weighted averaging, according to the Formula (1), over the whole constituent units i to n according to the molar ratio $m_i$ of the constituent unit i to calculate the chemical structure parameter P.

The value of the chemical structure parameter P itself is not particularly limited as long as the condition of the Formula (2) is satisfied, but from the viewpoint of satisfying the conditions of the Formula (2) in a case where the weight average molecular weight M of a specific (co)polymer is in a range that makes it relatively easy to manufacture and use, and from the viewpoint of facilitating satisfying the more preferable conditions of the following Formulae (3) and (4), and

the like, the value of the chemical structure parameter P is preferably 1.20 to 4.50, more preferably 2.10 to 4.20, still more preferably 2.80 to 4.00, particularly preferably 3.00 to 4.00, particularly preferably in the range of 3.40 to 4.00, and most preferably 3.50 to 3.85.

The chemical structure parameter P can be appropriately adjusted by selecting and adjusting the chemical structure of a specific (co)polymer, particularly the type and proportion of each constituent unit. For example, the value of the chemical structure parameter P can be increased by introducing a constituent unit having a tertiary or quaternary amine or ammonium salt and increasing the proportion thereof. In contrast, the value of the chemical structure parameter P can be lowered by introducing a constituent unit which do not have any amine, ammonium salt, carbonyl group, or sulfonyl group and increasing the proportion thereof.

From the viewpoint of increasing the value of the chemical structure parameter P, it is preferable that a specific (co)polymer has a constituent unit having a tertiary or quaternary amine or ammonium salt, and it is particularly preferable that a specific (co)polymer has a (di)allylamine-based constituent unit ($\alpha$) having a tertiary or quaternary amine or ammonium salt. The proportion of the (di)allylamine-based constituent unit ($\alpha$) having a tertiary or quaternary amine or ammonium salt to the whole constituent units of the specific (co)polymer is preferably 40 mol% or more, more preferably 50 to 100 mol%, more preferably 70 to 100 mol%, and particularly preferably 80 to 100 mol%.

Further, when a nonionic constituent unit is contained as a constituent unit other than the constituent unit having a tertiary or quaternary amine or ammonium salt, the proportion of the total of the constituent unit having a tertiary or quaternary amine or ammonium salt and the nonionic constituent unit to the whole constituent units of a specific (co)polymer is usually 50 mol% or more, preferably 70 to 100 mol%, more preferably 80 to 100 mol%, and particularly preferably 90 to 100 mol%.

The chemical structure parameter P and the weight average molecular weight M of a specific (co)polymer are in a relationship defined by the following Formula (2).

$$0.84 \leqq P^3/M^{1/4} \leqq 12.67 \quad \cdots (2)$$

That is, the ratio of the cube of the chemical structure parameter P to the 1/4 power of the weight average molecular weight M, $P^3/M^{1/4}$, is within a numerical range of 0.84 to 12.67.

When the chemical structure parameter P and the weight average molecular weight M of a specific (co)polymer are in the relationship defined by the above Formula (2), an agent for raising the melting temperature (Tm value) of a nucleic acid containing the specific (co)polymer according to this embodiment can effectively increase the Tm value of a hybridized double-stranded nucleic acid. By effectively raising the Tm value, for example, when there is no substance that affects the Tm value other than the agent for raising the Tm value of a nucleic acid of this embodiment, the difference between the Tm value of a hybridized double-stranded nucleic acid measured in the presence of the agent for raising the Tm value of a nucleic acid containing the specific (co)polymer according to the present embodiment and the Tm value of the hybridized double-stranded nucleic acid measured under the same condition except that the agent for raising a Tm value is absent, can be 20°C or more. The upper limit of a raised level of the Tm value is not particularly limited but is, for example, 50°C.

It is preferable that the chemical structure parameter P and the weight average molecular weight M of a specific (co)polymer are in a relationship defined by the following Formula (3).

$$2.37 \leqq P^3/M^{1/4} \leqq 6.92 \quad \cdots (3)$$

That is, it is preferable that the ratio of the cube of the chemical structure parameter P to the 1/4 power of the weight average molecular weight M, $P^3/M^{1/4}$, is preferably within a numerical range of 2.37 to 6.92.

When the chemical structure parameter P and the weight average molecular weight M of a specific (co)polymer are in the relationship defined by the above Formula (3), an agent for raising the melting temperature (Tm value) of a nucleic acid containing a specific (co)polymer according to this embodiment can more effectively raise the Tm value of a hybridized double-stranded nucleic acid. By further effectively raising the Tm value, for example, when there is no substance that affects the Tm value other than the agent for raising the Tm value of a nucleic acid of this embodiment, the difference between the Tm value of a hybridized double-stranded nucleic acid measured in the presence of an agent for raising the Tm value of a nucleic acid containing the specific (co)polymer of this embodiment and the Tm value of the hybridized double-stranded nucleic acid measured under the same condition except that the agent for raising a Tm value is absent, can be 30°C or more.

It is particularly preferable that the chemical structure parameter P and the weight average molecular weight M of a specific (co)polymer are in a relationship defined by the following Formula (4).

$$2.60 \leq P^3/M^{1/4} \leq 3.00 \quad \cdots (4)$$

That is, it is preferable that the ratio of the cube of the chemical structure parameter P to the 1/4 power of the weight average molecular weight M, $P^3/M^{1/4}$, is preferably within a numerical range of 2.60 to 3.00.

When the chemical structure parameter P and the weight average molecular weight M of a specific (co)polymer are in the relationship defined by the above Formula (4), an agent for raising the melting temperature (Tm value) of a nucleic acid containing the specific (co)polymer according to this embodiment can particularly effectively raise the Tm value of a hybridized double-stranded nucleic acid. By particularly effectively raising the Tm value, for example, when there is no substance that affects the Tm value other than the agent for raising the Tm value of a nucleic acid of this embodiment, the difference between the Tm value of a hybridized double-stranded nucleic acid measured in the presence of the agent for raising the Tm value of a nucleic acid containing the specific (co)polymer of this embodiment and the Tm value of the hybridized double-stranded nucleic acid measured under the same condition except that the agent for raising a Tm value is absent, can be 35°C or more.

As long as the conditions of the Formula (2) are satisfied, the weight average molecular weight M of a specific (co)polymer is not particularly limited, and a specific (co)polymer having an appropriate weight average molecular weight M suitable for relation with the chemical structure parameter P and for relation with the use form of an agent for raising the Tm value of a nucleic acid according to this embodiment, other components, and the like, may be obtained or polymerized.

From the viewpoint of ease of forming a reaction liquid with a nucleic acid or the like and performing polymerization in a practically acceptable time and cost, the weight average molecular weight M (Mw) of a specific (co)polymer is preferably 400 to 400000. The weight average molecular weight M (Mw) is more preferably 600 to 360000, still more preferably 700 to 300000, particularly preferably 3500 to 250000, particularly preferably 8000 to 240000, and most preferably 100000 to 200000.

The weight average molecular weight M (Mw) of a specific (co)polymer is measured by gel permeation chromatography (GPC method) using a liquid chromatograph. More specifically, it can be measured, for example, by the method described in Examples of the present application.

The weight average molecular weight M of a specific (co)polymer can be appropriately adjusted by adjusting the type and composition of monomers involved in polymerization, the temperature, time, and pressure in the polymerization step, the type and amount of a radical initiator and the like used in the polymerization step, and the like.

The rotational viscosity [η] of a specific (co)polymer is also not particularly limited and can be appropriately set in consideration of the use form, production cost, and the like of an agent for raising the Tm value of a nucleic acid of the present embodiment, but the rotational viscosity [η] is preferably 1.0 to 1500.0 mPa s (25°C), and particularly preferably 1.5 to 1000.0 mPa s (25°C), in 25.0% by mass of aqueous solution.

The rotational viscosity [η] can be measured by any method commonly used in the art, and can be measured by, for example, a digital B-type viscometer DV-3T manufactured by AMETEK Brookfield, Inc. The measurement can be performed using a ULA adaptor, typically at a liquid volume of 16 ml and a liquid temperature of 25°C.

The rotational viscosity [η] can also be appropriately adjusted by adjusting the dilution concentration during polymerization, the type and composition of monomers involved in polymerization, the temperature, time, and pressure in the polymerization step, the type and amount of a radical initiator and the like used in the polymerization step, and the like.

Other Constituent Units

A specific (co)polymer may have other constituent units in addition to a (di)allylamine-based constituent unit (α).

Examples of the other constituent units include various cationic constituent units other than a (di)allylamine-based constituent unit (α), various anionic constituent units, and various nonionic constituent units.

From the viewpoint of being able to select a constituent unit containing a carbonyl group or a sulfonyl group that can contribute to the chemical structure parameter P, it is preferable that the specific (co)polymer further comprises a nonionic constituent unit.

Anionic Constituent Unit

In this embodiment, a specific (co)polymer comprises an anionic constituent unit and thus becomes a so-called amphoteric polymer.

It is preferable that an anionic constituent unit in this embodiment has a structure represented by the following Structural Formula (III), (IV), or (V). In this case, a carbonyl group, that can contribute to the chemical structure parameter P, can be introduced into the structure of a specific (co)polymer.

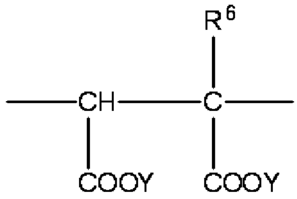

(III)

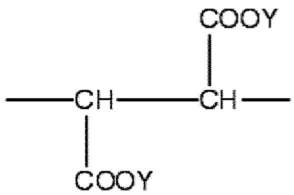

(IV)

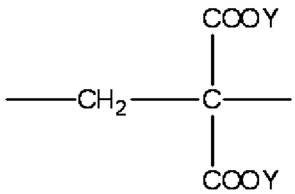

(V)

In the above Formula (III), $R^6$ represents hydrogen or a methyl group, and in the Formulae (III), (IV) and (V), Y represents hydrogen, Na, K, $NH_4$, 1/2Ca, 1/2Mg, 1/2Fe, 1/3Al or 1/3Fe independently for each carboxy group to be bonded.

The anionic constituent unit may have a structure that does not correspond to any of the Structural Formulae (III), (IV), and (V) as long as the anionic constituent unit has an anionic group such as a carboxyl group, and the anionic constituent unit may be, for example, a constituent unit derived from methacrylic acid, acrylic acid, or the like.

In order to adjust the positive charge of a (di)allylamine-based constituent unit ($\alpha$), it is preferable to comprise an anionic constituent unit when it is desired to adjust a raised amount of the Tm value to a desired level, but it is preferable not to comprise an anionic constituent unit from the viewpoint of increasing a raised amount of the Tm value.

When a specific (co)polymer comprises an anionic constituent unit, only one type of anionic constituent unit may be used alone, or a plurality of types of anionic constituent units having mutually different structures may be used in combination.

In a case where a specific (co)polymer has an anionic constituent unit, the content of the anionic constituent unit is not particularly limited, but the content may be appropriately set so as to obtain an appropriate chemical structure parameter P in relation to the type and amount of the (di)allylamine-based constituent unit ($\alpha$).

When an anionic constituent unit is derived from an unsaturated dicarboxylic acid, the molar ratio of the anionic constituent unit to a (di)allylamine-based constituent unit ($\alpha$) is preferably shown by the formula: (di)allylamine-based constituent unit ($\alpha$)/anionic constituent unit=1/0.1 to 0.1/1, and particularly preferably 1/0.5 to 2/1.

At least part of anionic constituent units may be derived from an unsaturated monocarboxylic acid such as acrylic acid or methacrylic acid. When an anionic constituent unit is derived from an unsaturated monocarboxylic acid, the ratio of (di)allylamine-based constituent unit ($\alpha$)/anionic constituent unit is preferably 2/1 to 0.1/1, and particularly preferably 1/1 to 0.5/1.

Nonionic Constituent Unit

In this embodiment, a specific (co)polymer comprises a nonionic constituent unit, and a constituent unit containing a carbonyl group or a sulfonyl group that can contribute to the chemical structure parameter P can be selected and introduced.

A nonionic constituent unit in this embodiment may be a constituent unit derived from a nonionic monomer that can be copolymerized with a monomer from which a (di)allylamine-based constituent unit ($\alpha$) is derived, and is not otherwise particularly limited; however, as a nonionic constituent unit, a constituent unit derived from a methacrylic acid ester-based monomer, an acrylic ester-based monomer, a methacrylamide-based monomer, an acrylamide-based monomer, sulfur dioxide, or the like can be preferably employed. More specific examples of the nonionic constituent unit include constituent units derived from methyl methacrylate, ethyl methacrylate, butyl methacrylate, methyl acrylate, ethyl

acrylate, butyl acrylate, methacrylamide, N-methyl methacrylamide, dimethyl methacrylamide, N-(3-dimethylaminopropyl) methacrylamide, acrylamide, dimethyl acrylamide, hydroxyethyl acrylamide, dimethylaminopropyl acrylamide, a dimethylaminopropyl acrylamide methyl chloride quaternary salt, acryloylmorpholine, isopropyl acrylamide, 4-t-butylcyclohexyl acrylate, and sulfur dioxide. Among them, a constituent unit derived from sulfur dioxide, an acrylamide monomer, or the like is particularly preferable.

A nonionic constituent unit can usually be introduced into a polymer by using a nonionic monomer as the monomer. When a specific (co)polymer has a nonionic constituent unit, the content of the nonionic constituent unit is not particularly limited, and a suitable amount thereof varies depending on the type of a nonionic constituent unit, but those skilled in the art could appropriately set the content so as to obtain an appropriate chemical structure parameter P in relation to the type and amount of the (di)allylamine-based constituent unit ($\alpha$). For example, by introducing a constituent unit derived from sulfur dioxide, a sulfonyl group that can contribute to the chemical structure parameter P can be introduced into the structure of a specific (co)polymer.

For example, the molar ratio of a (di)allylamine-based constituent unit ($\alpha$) to a nonionic constituent unit is preferably shown by the formula: (di)allylamine-based constituent unit ($\alpha$)/nonionic constituent unit=1/0.05 to 1/0.05, and particularly preferably 1/0.1 to 0.1/1.

When a nonionic constituent unit is derived from a methacrylic acid ester-based monomer, an acrylic acid ester-based monomer, a methacrylamide-based monomer, or an acrylamide-based monomer, the ratio is preferably 1/0.05 to 1/0.5, and particularly preferably 1/0.1 to 1/1.

In a case where a nonionic constituent unit is derived from sulfur dioxide, the above ratio is preferably 1/0.1 to 1/1.5, and particularly preferably 1/0.25 to 1/1.

Method for Producing Specific (Co)Polymer

A method for producing a specific (co)polymer is not particularly limited, and the specific (co)polymer can be produced by a method known in the art, but for example, a specific (co)polymer can be produced by copolymerizing a (di)allylamine-based monomer having a structure corresponding to the (di)allylamine-based constituent unit ($\alpha$) and a monomer corresponding to another constituent unit such as a nonionic constituent unit as desired.

A solvent for use in (co)polymerizing a (di)allylamine-based monomer or the like having a structure corresponding to the (di)allylamine-based constituent unit ($\alpha$) is not particularly limited, and may be an aqueous solvent or an organic solvent such as alcohol, ether, sulfoxide, or amide, but the solvent is preferably an aqueous solvent.

In the case of (co)polymerizing a (di)allylamine-based monomer or the like having a structure corresponding to a (di)allylamine-based constituent unit ($\alpha$), the monomer concentration varies depending on the type of the monomer and depending on the type of the solvent in which the (co)polymerization is performed; however the monomer concentration is usually 10 to 75% by mass in an aqueous solvent. This copolymerization reaction is usually a radical polymerization reaction, and is performed in the presence of a radical polymerization catalyst. The type of the radical polymerization catalyst is not particularly limited, and preferred examples of the radical polymerization catalyst include a peroxide such as t-butyl hydroperoxide, a persulfate such as ammonium persulfate, sodium persulfate, and potassium persulfate; and a water-soluble azo compound such as an azobis-based compound, and a diazo-based compound.

The amount of a radical polymerization catalyst to be added is commonly 0.1 to 20 mol%, and preferably 1.0 to 10 mol% based on the whole monomers. The polymerization temperature is commonly 0 to 100°C and preferably 5 to 80°C, and the polymerization time is commonly 1 to 150 hours and preferably 5 to 100 hours. As to the polymerization atmosphere, the polymerization can be performed in an atmosphere of an inert gas such as nitrogen although even the air does not cause a significant problem in polymerization.

Specific Monomer

A specific monomer, which is another form of a specific compound, is a (di)allylamine-based compound ((di)allylamine-based monomer) which comprises:

at least one type of (di)allylamine-based structure (constituent unit) selected from the group consisting of a monomer structure (A2) which is a structure represented by General Formula (I-c) or an acid addition salt thereof (hereinafter, also referred to as a "constituent unit (A2)"):

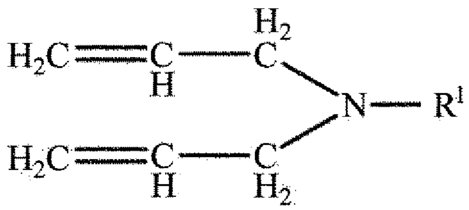

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,

a monomer structure (B2) which is a structure represented by General Formula (I-f) (hereinafter, also referred to as "constituent unit (B2)"):

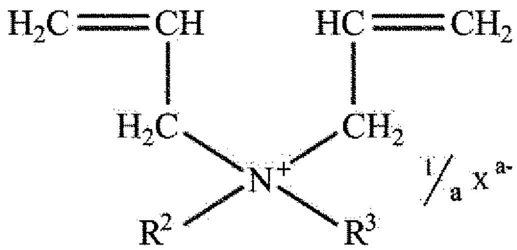

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and

a monomer structure (C2) which is a structure represented by General Formula (I-h), or an acid addition salt thereof (hereinafter, also referred to as "constituent unit (C2)"):

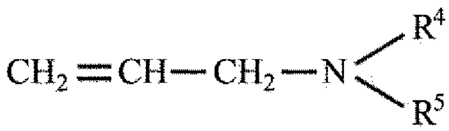

(I-h)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms, and

in which its chemical structure parameter P calculated by the following Formula (1) and its average molecular weight M are in a relationship satisfying the following Formula (2):

$$P = \frac{\sum_{i=1}^{n} m_i\left(\sum_{j=1}^{4} j \times a_{ij} + o_i + 2 \times s_i\right)}{\sum_{i=1}^{n} m_i} \quad \cdots \quad (1)$$

wherein n represents the number of constituent (unit) species constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and $s_i$ represents the number of sulfonyl groups in the constituent unit i.

$$0.84 \leq P^3/M^{1/4} \leq 12.67 \cdots (2)$$

Since a specific monomer is a monomer, in the above Formula (1), n=1, and thus $m_i$ of the denominator and $m_i$ of the numerator cancel each other. Further, since only one amino group is present in all of the structures represented by Formulae (I-c), (I-f), and (I-h), $a_{ij}$ is 1. Therefore, Formula (1) can be simplified to the following Formula (1').

$$P=j+o+2\times s \ldots (1')$$

wherein j represents the class number of an amino group in the monomer structure, o represents the number of carbonyl groups in the monomer structure, and s represents the number of sulfonyl groups in the monomer structure.

The value of the chemical structure parameter P of a specific monomer itself is not particularly limited as long as the conditions of the Formula (2) are satisfied, but from the viewpoint of satisfying the conditions of the Formula (2) in a realistic relationship with the molecular weight M of the specific monomer, the chemical structure parameter P is preferably 2.00 to 4.00, and particularly preferably 2.00 to 3.00.

The chemical structure parameter P of the specific monomer can be appropriately adjusted by selecting and adjusting the chemical structure of the specific monomer, particularly the class number of amino groups, and the presence or absence and number of carbonyl groups and/or sulfonyl groups.

From the viewpoint of increasing the value of the chemical structure parameter P, it is preferable that the specific monomer has a secondary or tertiary amine or ammonium salt.

With regard to the specific monomer, the molecular weight M in the Formula (2) is the molecular weight of the specific monomer molecule, and can be calculated from the chemical structure of the specific monomer.

The molecular weight M of the specific monomer is not particularly limited as long as the condition of the Formula (2) is satisfied, but from the viewpoint of easily satisfying the conditions of the Formula (2) in relation to the chemical structure parameter P, the ease of handling, and the like, the molecular weight M is preferably 100 to 700, and particularly preferably 120 to 300.

Similarly to a specific (co)polymer, it is also preferable for the specific monomer that the chemical structure parameter P and the molecular weight M are in a relationship satisfying the above Formula (3), and more preferably in a relationship satisfying the above Formula (4).

<u>Monomer Structure (A2)</u>

A monomer structure (A2) (constituent unit (A2)), which is an optional structure of a specific monomer, is a structure represented by the following General Formula (I-c) or an acid addition salt thereof.

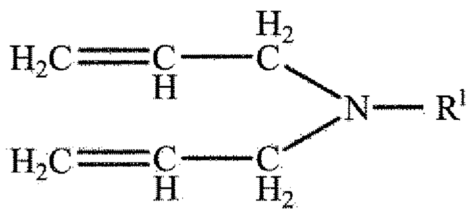

(I-c)

In Formula (I-c), $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms. Preferred examples of $R^1$ are the same as those described above for the constituent unit (A1).

In the case where the monomer structure (A2) is an acid addition salt having a structure represented by the General Formula (I-c), preferred examples of the acid addition salt are also the same as those described above for the constituent unit (A1).

Specific examples of a preferred monomer structure (A2) include diallylmethylamine hydrochloride, diallylamine, and diallylamine hydrochloride.

Monomer Structure (B2)

A monomer structure (B2) (constituent unit (B2)), which is an optional structure of a specific monomer, is a structure represented by the following General Formula (I-f).

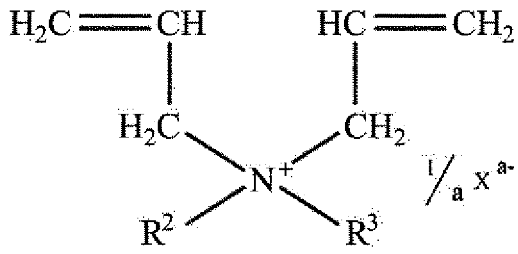

(I-f)

In the formula, $R^2$ and $R^3$ each independently represent a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion.

Preferred examples of $R^2$ and $R^3$ are the same as those described above for the constituent unit (B1).

Preferred examples of the counter ion $X^{a-}$ are also the same as those described above for the constituent unit (B 1).

Specific examples of a preferred monomer structure (B2) include diallyldinonadecylammonium chloride and diallyldiicosylammonium chloride.

Monomer Structure (C2)

A monomer structure (C2) (constituent unit (C2)), which is an optional structure of a specific monomer, is a structure represented by the following General Formula (I-h) or an acid addition salt thereof.

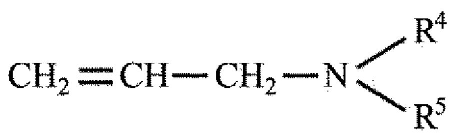

(I-h)

In the formula, $R^4$ and $R^5$ independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms.

Preferred examples of $R^4$ and $R^5$ are the same as those described above for the constituent unit (C1).

In the case where the monomer structure (C2) is an acid addition salt having a structure represented by the General Formula (I-h), preferred examples of the acid addition salt are also the same as those described above for the constituent unit (C1).

Specific examples of a preferred monomer structure (C2) include N,N-dimethylallylamine and methoxycarbonylated allylamine.

The specific (co)polymer and the specific monomer described above can be comprehensively combined to specify a specific compound used in the present invention.

That is, a specific compound according to the present invention is a (di)allylamine-based compound which comprises:

at least one type of (di)allylamine-based constituent unit selected from the group consisting of
a constituent unit (A) having a structure represented by General Formula (I-a), General Formula (I-b), or General Formula (I-c), or a structure of an acid addition salt thereof:

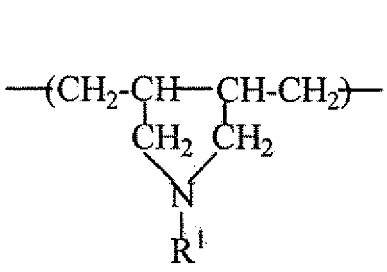

(I-a)

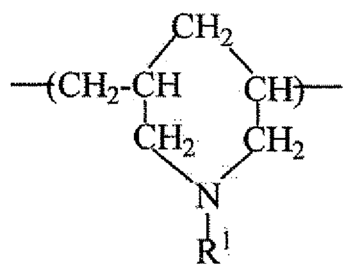

(I-b)

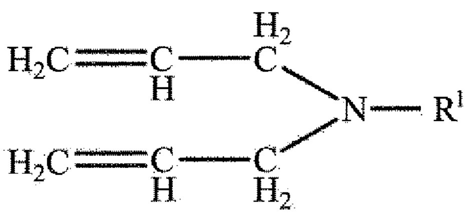

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,
a constituent unit (B) having a structure represented by General Formula (I-d), General Formula (I-e), or General Formula (I-f):

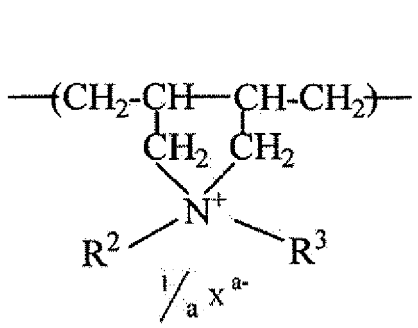

(I-d)

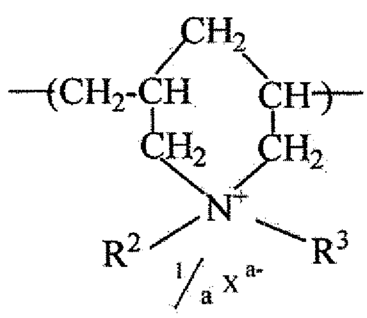

(I-e)

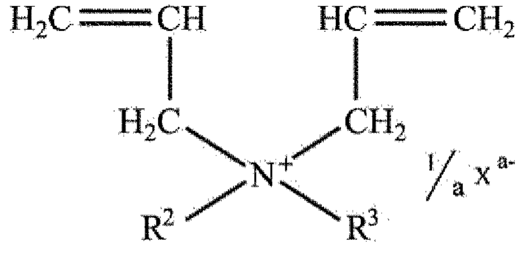

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and
a constituent unit (C) having a structure represented by General Formula (I-g) or General Formula (1-h), or a structure of an acid addition salt thereof:

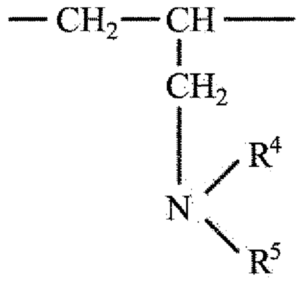

(I-g)

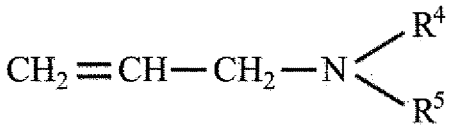

(I-h)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms, and
in which its chemical structure parameter P calculated by the following Formula (1) and its molecular weight M are in a relationship satisfying the following Formula (2):

$$P = \frac{\sum_{i=1}^{n} m_i \left( \sum_{j=1}^{4} j \times a_{ij} + o_i + 2 \times s_i \right)}{\sum_{i=1}^{n} m_i} \quad \cdots \quad (1)$$

wherein n represents the number of constituent units constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and $s_i$ represents the number of sulfonyl groups in the constituent unit i.

$$0.84 \leqq P^3/M^{1/4} \leqq 12.67 \quad \cdots \quad (2)$$

Here, the constituent unit (A) is a generic term for the above-described constituent unit (A1) and constituent unit (A2) (monomer structure (A2)), the constituent unit (B) is a generic term for the above-described constituent unit (B1) and constituent unit (B2) (monomer structure (B2)), and the constituent unit (C) is a generic term for the above-described constituent unit (C1) and constituent unit (C2) (monomer structure (C2)).

Applications and the Like

An agent for raising the Tm value of a nucleic acid according to the present invention brings about a significant rise in the Tm value with respect to a double-stranded nucleic acid. Due to the characteristics of the Tm value, a difference in the width of the Tm value rise upon addition of an agent for raising the Tm value of a nucleic acid is expected to occur between a double strand of which strands are fully complementary and a double strand of which strands have a mismatch and are not fully complementary. Therefore, it is expected that a mutation to a standard sequence can be detected with higher sensitivity using the Tm value. Further, when a nucleic acid amplification cycle is performed in a solution containing an agent for raising the Tm value of a nucleic acid of the present invention, a difference in amplification efficiency is expected to be greater between a double strand of which strands are fully complementary and a double strand of which strands have a mismatch and are not fully complementary. Therefore, it is expected to more efficiently detect mutations with high sensitivity by nucleic acid amplification. Further, since the annealing temperature can be increased in the

nucleic acid amplification reaction, the dependency on GC content can be reduced when designing a primer to be used, and a greater degree of freedom in primer design is expected.

The method for nucleic acid amplification is not particularly limited, and examples thereof include, but are not limited to, a PCR method, a TMA method, a LAMP method, an ICAN method, an SDA method, an LCR method, a NASBA method, an HDA method, an RCA method, and an RPA method.

Further, a rise in the Tm value means stabilization of a double-stranded nucleic acid. Therefore, a Tm raising agent of the present invention can be suitably used to stabilize a nucleic acid pharmaceutical product or a sample to be subjected to nucleic acid analysis.

Specific examples of a sample to be subjected to nucleic acid analysis include samples for medicine and clinical diagnosis, development and evaluation of pharmaceutical compositions, food analysis, monitoring of food production, agriculture, environmental analysis, and many research fields, but the examples are not limited to these.

Therefore, an embodiment of the present invention provides a method for stabilizing a nucleic acid which comprises adding an agent for raising the Tm value of a nucleic acid of the present invention into a solution containing a nucleic acid, as well as an aqueous solution containing the agent for raising a Tm value of a nucleic acid of the present invention together with a nucleic acid.

## EXAMPLES

Hereinafter, the present invention will be described in more detail with reference to Examples. However, the technical scope of the present invention is not limited by these Examples in any sense.

### Measurement of weight average molecular weight M and determination of structural parameter P

### (Weight Average Molecular Weight M)

The weight average molecular weight M (Mw) of a polymer was measured by gel permeation chromatography (GPC method) using a Hitachi L-6000 type high performance liquid chromatograph.

An eluent channel pump used was Hitachi L-6000, a detector used was Shodex RI-101 differential refractive index, and columns used were Shodex Asahipak water-based gel filtration type GS-220HQ (exclusion limit molecular weight 3000) and GS-620HQ (exclusion limit molecular weight 2 million) connected in series. A sample was prepared at a concentration of 0.5 g/100 ml with an eluent, and 20 μL thereof was used. As an eluent, a 0.4 mol/L aqueous solution of sodium chloride was used. The column temperature was 30°C and the flow rate was 1.0 ml/min.

A calibration curve was obtained using polyethylene glycols having molecular weights of 106, 194, 440, 600, 1470, 4100, 7100, 10300, 12600, 23000, 48290, 66200, 117900, 205500, and the like as a standard substance, and the weight average molecular weight (Mw) of the polymer was obtained on the basis of the calibration curve.

### (Structural Parameter P)

The structural parameter P of the (co)polymer or monomer contained in the agent for raising the Tm value of a nucleic acid in each Example or Comparative Example was determined by the following formula.

$$P = \frac{\sum_{i=1}^{n} m_i \left( \sum_{j=1}^{4} j \times a_{ij} + o_i + 2 \times s_i \right)}{\sum_{i=1}^{n} m_i}$$

In the formula, n represents the number of constituent units constituting the (co)polymer or monomer, $m_i$ represents a molar ratio of a constituent unit i, $a_{ij}$ represents the number of j-class amines (or ammonium salts) in the constituent unit i, $o_i$ represents the number of carbonyl groups in the constituent unit i, and $s_i$ represents the number of sulfonyl groups in the constituent unit i.

### [Example 1]

### 1-1. Polymer Aqueous Solution (Agent for Raising Tm Value of Nucleic Acid)

As an agent for raising the Tm value of a nucleic acid, prepared was 20.0 μL of a polymer aqueous solution having a pH of 7.0 and containing 10.0% by mass of a diallyldimethylammonium chloride acrylamide copolymer that was obtained by copolymerizing diallyldimethylammonium chloride (monomer 1) and acrylamide (monomer 2) under

the following polymerization conditions 1 and had a molar ratio of 8:1 between a constituent unit 1 derived from the monomer 1 and a constituent unit 2 derived from the monomer 2 and a weight average molecular weight M of 180000 as determined by GPC measurement. The structural parameter P was 3.78 and $P^3/M^{1/4}$ was 2.62.

Polymerization conditions 1: Into a 300-ml four-neck flask equipped with a thermometer, a stirrer, and a cooling pipe, 119.39 g (0.48 mol) of 65.0% diallyldimethylammonium chloride, 4.26 g (0.06 mol) of acrylamide, and 149.24 g of distilled water were charged, and the internal temperature was raised to 50°C. 15% by mass of V-50 (2,2'-azobis(2-methylpropionamidine)dihydrochloride) aqueous solution was added at such an amount that the amount of V-50 in the aqueous solution was 0.2% by mass to the whole amount of monomers to start the polymerization. The V-50 aqueous solution was added at such an amount that the amount of V-50 was 0.2% by mass to the whole amount of monomers after 22 and 25 hours, respectively, and 0.3% by mass to the whole amount of monomers after 28, 46, and 53 hours, respectively, and the mixture was reacted for 72 hours.

1-2. Nucleic Acid Molecule

In order to evaluate the rise level of the Tm value by adding the agent for raising the Tm value of a nucleic acid, a 40-mer DNA oligomer derived from a part of Exon2 of the sense strand of wild-type KRAS gene and a 40-mer DNA oligomer of an antisense strand having a sequence complementary thereto were used. The base sequences of both nucleic acid molecules are as follows.

[Table 1]

| | |
|---|---|
| 40-mer DNA oligomer derived from a part of Exon 2 of sense strand of wild-type KRAS gene | GTGGTAGTTGC,AGCTGGTGGCGTAGGCAAGAGTGCCTTGA (SEQ ID NO: 1) |
| 40-mer DNA oligomer of antisense strand having sequence complimentary to DNA oligomer of the sense strand | TCAAGGCACTCTTGCCTACGCCACCAGCTCCAACTACCAC (SEQ ID NO:2) |

1-3. Preparation of Reaction Mixture

The following reaction mixtures 1 and 2 were prepared using the above-described polymer aqueous solution and nucleic acid molecules (the concentration of the (co)polymer in the reaction mixture 2 was 1.0% by mass).

[Table 2] Reaction Mixture 1

| Component | Content |
|---|---|
| Aqueous solution of 100 μM of sense strand DNA oligomer | 8.5 μL |
| Aqueous solution of 500 μM of antisense strand DNA oligomer | 8.5 μL |
| 25 μM fluorescent dye solution (manufactured by Biotium, Inc., trade name: EvaGreen) | 1.0 μL |
| Nuclease-free water | 2.0 μL |

Reaction Mixture 2

| Component | Content |
|---|---|
| Aqueous solution of 100 μM of sense strand DNA oligomer | 8.5 μL |
| Aqueous solution of 500 μM of antisense strand DNA oligomer | 8.5 μL |
| 25 μM fluorescent dye solution (manufactured by Biotium, Inc., trade name: EvaGreen) | 1.0 μL |
| 10.0% by mass of polymer aqueous solution | 2.0 μL |

1-4. Measurement of Tm Rise Value

The obtained reaction mixtures 1 and 2 were set in StepOnePlus Real-Time PCR System (manufactured by

Thermo Fisher Scientific, Inc.), heated at 95°C for 15 seconds, and then kept warm at 40°C for 1 minute. Next, while performing fluorescence measurement every 0.3 seconds, the temperature was raised to 95°C to obtain a DNA melting curve, and then the reaction mixtures were held at 95°C for 15 seconds.

The Tm value was determined from the obtained DNA melting curve by a differentiation method, and the Tm value of the reaction mixture 1 was subtracted from the Tm value of the reaction mixture 2 to determine the Tm rise value. The Tm value of the reaction mixture 1 was 58.00°C, and the Tm value of the reaction mixture 2 was 93.30°C. As a result, the Tm rise value was 35.3°C.

<Example 2>

The Tm rise value was determined in the same manner as in Example 1, except that an aqueous solution that contained a diallyldimethylammonium chloride-acrylamide copolymer obtained by copolymerizing diallyldimethylammonium chloride (monomer 1) and acrylamide (monomer 2) under the following polymerization conditions 2, and had a molar ratio of the constituent unit 1 derived from the monomer 1 to the constituent unit 2 derived from the monomer 2 of 8:1 and a weight average molecular weight M obtained by GPC measurement of 10000, was used as the agent for raising a Tm value of a nucleic acid. The structural parameter P of this copolymer was 3.78, and $P^3/M^{1/4}$ was 5.40. Further, the Tm rise value was 33.3°C.

Polymerization conditions 2: Into a 300-ml four-neck flask equipped with a thermometer, a stirrer, and a cooling pipe, 124.37 g (0.50 mol) of 65.0% diallyldimethylammonium chloride, 4.44 g (0.06 mol) of acrylamide, 0.85 g of sodium hypophosphite, and 84.39 g of distilled water were charged, and the internal temperature was raised to 50°C. 28.5% by mass of an ammonium persulfate aqueous solution was added at such an amount that the amount of ammonium persulfate in the aqueous solution was 0.2% by mass to the whole amount of monomers, and the polymerization was started. The ammonium persulfate aqueous solution was added at such an amount that the amount of ammonium persulfate was 0.3% by mass to the whole amount of monomers after 4 hours, 0.5% by mass to the whole amount of monomers after 23 hours, and 1.0% by mass to the whole amount of monomers after 28 hours, and the mixture was reacted for 48 hours.

<Example 3>

The Tm rise value was determined in the same manner as in Example 1, except that an aqueous solution containing polydiallyldimethylammonium chloride (product name: PAS-H-1L, manufactured by Nittobo Medical Co., Ltd.), which was obtained by polymerizing diallyldimethylammonium chloride (monomer corresponding to the constituent unit 1) and had a weight average molecular weight M of 8500 as obtained by GPC measurement, was used as the agent for raising a Tm value of a nucleic acid. The structural parameter P of this copolymer was 4.00, and $P^3/M^{1/4}$ was 6.67. Further, the Tm rise value was 33.3°C.

<Example 4>

The Tm rise value was determined in the same manner as in Example 1, except that an aqueous solution containing polydiallyldimethylammonium chloride (product name: PAS-H-5L, manufactured by Nittobo Medical Co., Ltd.), which was obtained by polymerizing diallyldimethylammonium chloride (monomer corresponding to the constituent unit 1) and had a weight average molecular weight M of 30000 as obtained by GPC measurement, was used as the agent for raising a Tm value of a nucleic acid. The structural parameter P of this copolymer was 4.00, and $P^3/M^{1/4}$ was 4.86. Further, the Tm rise value was 33.5°C.

<Example 5>

The Tm rise value was determined in the same manner as in Example 1, except that an aqueous solution that contained a diallyldimethylammonium chloride-sulfur dioxide copolymer (product name: PAS-A-5, manufactured by Nittobo Medical Co., Ltd.) obtained by copolymerizing diallyldimethylammonium chloride (monomer 1) and sulfur dioxide (monomer 2), and had a molar ratio of the constituent unit 1 derived from the monomer 1 to the constituent unit 2 derived from the monomer 2 of 1:1 and a weight average molecular weight M obtained by GPC measurement of 4000, was used as the agent for raising a Tm value of a nucleic acid. The structural parameter P of this copolymer was 3.00, and $P^3/M^{1/4}$ was 3.40. Further, the Tm rise value was 33.8°C.

<Example 6>

The Tm rise value was determined in the same manner as in Example 1, except that an aqueous solution that

contained a diallylmethylamine hydrochloride-sulfur dioxide copolymer (product name: PAS-2201CL, manufactured by Nittobo Medical Co., Ltd.) obtained by copolymerizing diallylmethylamine hydrochloride (monomer 1) and sulfur dioxide (monomer 2), and had a molar ratio of the constituent unit 1 derived from the monomer 1 to the constituent unit 2 derived from the monomer 2 of 1:1 and a weight average molecular weight M obtained by GPC measurement of 3000, was used as the agent for raising a Tm value of a nucleic acid. The structural parameter P of this copolymer was 2.50, and $P^3/M^{1/4}$ was 2.11. Further, the Tm rise value was 24.0°C.

<Example 7>

The Tm rise value was determined in the same manner as in Example 1, except that an aqueous solution that contained an allylamine-dimethylallylamine copolymer obtained by copolymerizing allylamine (monomer 1) and dimethylallylamine (monomer 2) under the following polymerization conditions 3, and had a molar ratio of the constituent unit 1 derived from the monomer 1 to the constituent unit 2 derived from the monomer 2 of 1:1 and a weight average molecular weight M obtained by GPC measurement of 1000, was used as the agent for raising a Tm value of a nucleic acid. The structural parameter P of this copolymer was 2.00, and $P^3/M^{1/4}$ was 1.42. Further, the Tm rise value was 24.6°C.

Polymerization conditions 3: Into a 1-L four-neck flask equipped with a thermometer, a stirrer, and a cooling pipe, 209.67 g (1.3 mol) of 58.01% by mass of allylamine hydrochloride and 248.51 g (1.3 mol) of 63.61% by mass of dimethylallylamine hydrochloride were charged, and the temperature was raised to 60°C. Initiator V-50 (2,2'-azobis(2-methylpropionamidine) dihydrochloride) was added in three portions at such an amount that the amount of V-50 was 12 mol% to the whole amount of monomers, and polymerization was performed for 72 hours. Thereafter, thermal decomposition treatment was performed at 60°C for 48 hours. Thereafter, 449.28 g (2.81 mol) of sodium hydroxide having a concentration of 25% by mass was added under cooling at 30°C or lower, and the mixture was reacted at 40°C for 24 hours. Thereafter, demonomerization (50°C, 3 hours) by an evaporator was performed. After the demonomerization, the concentration was adjusted to 15%, and desalination by electrodialysis (ended in about 3 hours, 1 hour after conductivity completely decreased) was performed.

<Example 8>

The Tm rise value was determined in the same manner as in Example 1, except that an aqueous solution containing 50 mol% of ureated polyallylamine that contained allylamine (constituent unit 1) and ureated allylamine (constituent unit 2) obtained under the following reaction conditions 1 and had a molar ratio of the constituent unit 1 to the constituent unit 2 of 1:1, and a weight average molecular weight M obtained by GPC measurement of 15000, was used as the agent for raising a Tm value of a nucleic acid. The structural parameter P of this copolymer was 2.50, and $P^3/M^{1/4}$ was 1.41. Further, the Tm rise value was 22.8°C.

Reaction conditions 1: Into a 10-L four-neck flask equipped with a thermometer, a stirrer, and a cooling pipe, 1.88 kg (10.00 mol) of 49.77% by mass of allylamine hydrochloride polymer (product name: PAA-HCl-3L, manufactured by Nittobo Medical Co., Ltd.) and 2.21 kg of diluting water were charged. Thereafter, the temperature was raised to 50°C, 4.33 kg (5.00 mol) of a 7.50% by mass sodium cyanate aqueous solution was added dropwise, and the mixture was reacted overnight. Thereafter, 800 g (5.00 mol) of sodium hydroxide having a concentration of 25% by mass was added under cooling at 30°C or lower, and demonomerization (65°C, 20 hours) was performed under reduced pressure. After the demonomerization, the concentration was adjusted to 15%, and desalination by electrodialysis (ended in about 20 hours, 1 hour after conductivity completely decreased) was performed.

<Example 9>

The Tm rise value was determined in the same manner as in Example 1, except that an aqueous solution containing polydiallylamine (product name: PAS-21, manufactured by Nittobo Medical Co., Ltd.) obtained by polymerizing diallylamine (constituent unit 1) and having a weight average molecular weight M obtained by GPC measurement of 5000, was used as the agent for raising a Tm value of a nucleic acid. The structural parameter P of this copolymer was 2.00, and $P^3/M^{1/4}$ was 0.95. Further, the Tm rise value was 21.9°C.

<Comparative Example 1>

The Tm rise value was determined in the same manner as in Example 1, except that an aqueous solution containing 50 mol% of methoxycarbonylated polyallylamine that contained allylamine (constituent unit 1) and methoxycarbonylated allylamine (constituent unit 2) obtained under the following reaction conditions 2 and had a molar ratio of the constituent unit 1 to the constituent unit 2 of 1:1, and a weight average molecular weight M obtained by GPC measurement of 15000, was used as the agent for raising a Tm value of a nucleic acid. The structural parameter P of

this copolymer was 2.00, and $P^3/M^{1/4}$ was 0.72. Further, the Tm rise value was 15.2.

Reaction conditions 2: Into a 10-L four-neck flask equipped with a thermometer, a stirrer, and a cooling pipe, 3.81 kg (10.00 mol) of 15.00% by mass of an allylamine polymer (product name: PAA-15C, manufactured by Nittobo Medical Co., Ltd.) was charged. Thereafter, the temperature was raised to 40°C, 0.45 kg (5.00 mol) of 99.00% by mass dimethyl carbonate was added dropwise thereto, and the mixture was reacted overnight at 45°C. Thereafter, methanol as a by-product was removed by pressure reduction. (50°C, 20 hours) was performed. Thereafter, the concentration was adjusted to 15%, and a 50% methoxycarbonylated polyallylamine aqueous solution was obtained.

<Comparative Example 2>

The Tm rise value was determined in the same manner as in Example 1, except that an aqueous solution containing polydiallylamine hydrochloride (product name: PAS-21CL, manufactured by Nittobo Medical Co., Ltd.) that was obtained by polymerizing diallylamine hydrochloride (constituent unit 1) and had a weight average molecular weight M obtained by GPC measurement of 50000, was used as the agent for raising a Tm value of a nucleic acid. The structural parameter P of this copolymer was 2.00, and $P^3/M^{1/4}$ was 0.53. Further, the Tm rise value was 18.1°C.

<Comparative Example 3>

The Tm rise value was determined in the same manner as in Example 1, except that an aqueous solution that contained an allylamine hydrochloride-diallylamine hydrochloride copolymer obtained by copolymerizing allylamine hydrochloride (constituent unit 1) and diallylamine hydrochloride (constituent unit 2) under the following polymerization conditions 4, and had a molar ratio of the constituent unit 1 to the constituent unit 2 of 1:19 and a weight average molecular weight M obtained by GPC measurement of 40000, was used as the agent for raising a Tm value of a nucleic acid. The structural parameter P of this copolymer was 1.95, and $P^3/M^{1/4}$ was 0.52. Further, the Tm rise value was 18.2°C.

Polymerization conditions 4: Into a 1-L four-neck flask equipped with a thermometer, a stirrer, and a cooling pipe, 10.63 g (0.065 mol) of 57.22% by mass of allylamine hydrochloride, 253.02 g (1.235 mol) of 65.22% by mass of diallylamine hydrochloride, and 31.35 g (amount to obtain a concentration of 58% by mass) of diluting water were charged, and the temperature was raised to 60°C. 28.5% by mass of an ammonium persulfate aqueous solution was added at such an amount that the amount of ammonium persulfate in the aqueous solution was 0.25% by mass to the whole amount of monomers, and the polymerization was started. After 3, 5, and 21 hours, 0.25% by mass was added, and after 23, 25, 27, and 29 hours, 0.50% by mass was added. The mixture was further reacted overnight.

<Comparative Example 4>

The Tm rise value was determined in the same manner as in Example 1, except that an aqueous solution containing a monomer (molecular weight M: 161.5) of diallyldimethylammonium chloride (constituent unit 1) was used as the agent for raising a Tm value of a nucleic acid. The structural parameter P of this monomer was 4.00, and $P^3/M^{1/4}$ was 17.95. Further, the Tm rise value was 15.4°C.

The results obtained in Examples and Comparative Examples are summarized below.

[Table 3]

| | Constituent unit 1 | Constituent unit 2 | Constituent unit 1: constituent unit 2 | Weight average molecular weight M | Structure Parameter P | $P^3/M^{1/4}$ | Tm rise value (°C) |
|---|---|---|---|---|---|---|---|
| Example 1 | Diallyldimethylammonium chloride | Acrylamide | 8:1 | 180000 | 3.78 | 2.62 | 35.3 |
| Example 2 | Diallyldimethylammonium chloride | Acrylamide | 8:1 | 10000 | 3.78 | 5.40 | 33.3 |
| Example 3 | Diallyldimethylammonium chloride | - | - | 8500 | 4.00 | 6.67 | 33.3 |

(continued)

| | Constituent unit 1 | Constituent unit 2 | Constituent unit 1: constituent unit 2 | Weight average molecular weight M | Structure Parameter P | $P^3/M^{1/4}$ | Tm rise value (°C) |
|---|---|---|---|---|---|---|---|
| Example 4 | Diallyldimethylammonium chloride | - | - | 30000 | 4.00 | 4.86 | 33.5 |
| Example 5 | Diallyldimethylammonium chloride | Sulfur dioxide | 1:1 | 4000 | 3.00 | 3.40 | 33.8 |
| Example 6 | Diallylmethyl amine hydrochloride | Sulfur dioxide | 1:1 | 3000 | 2.50 | 2.11 | 24.0 |
| Example 7 | Allylamine | Dimethylallyl amine | 1:1 | 1000 | 2.00 | 1.42 | 24.6 |
| Example 8 | Allylamine | Ureated allylamine | 1:1 | 15000 | 2.50 | 1.41 | 22.8 |
| Example 9 | Diallylamine | - | - | 5000 | 2.00 | 0.95 | 21.9 |
| Comparative Example 1 | Allylamine | Methoxycarbonylated polyallylamine | - | 15000 | 2.00 | 0.72 | 15.2 |
| Comparative Example 2 | Diallylamine hydrochloride | - | - | 50000 | 2.00 | 0.53 | 18.1 |
| Comparative Example 3 | Allylamine hydrochloride | Diallylamine hydrochloride | 1:19 | 40000 | 1.95 | 0.52 | 18.2 |
| Comparative Example 4 | Diallyldimethylammonium chloride | - | - | 161.5 | 4.00 | 17.95 | 15.4 |

In each Example, an aqueous solution containing a (di)allylamine compound having a $P^3/M^{1/4}$ of 0.95 to 6.67 was used as the agent for raising a Tm value of a nucleic acid, and the Tm rise value was 20°C or more. In particular, in Examples 1 to 5 in which an aqueous solution containing a (di)allylamine compound having a $P^3/M^{1/4}$ of 3.40 to 6.67 was used, the Tm rise value was 33°C or more. On the other hand, in Comparative Examples 1 to 3 in which an aqueous solution containing a (di)allylamine compound having a $P^3/M^{1/4}$ of 0.72 or less was used, the Tm rise value was less than 20°C. Further, in Comparative Example 4 in which a monomer of diallyldimethylammonium chloride having a $P^3/M^{1/4}$ of 17.95 was used, the Tm rise value was 15.4°C, and this value was less than that in Examples 3 and 4 in which a polymer of diallyldimethylammonium chloride was used.

## Claims

**1.** An agent for use in raising a melting temperature (Tm value) of a nucleic acid, comprising

a (di)allylamine-based compound, wherein the (di)allylamine-based compound comprises: at least one type of (di)allylamine-based constituent unit selected from the group consisting of
a constituent unit (A) having a structure represented by General Formula (I-a), General Formula (I-b), or General Formula (I-c), or a structure of an acid addition salt thereof:

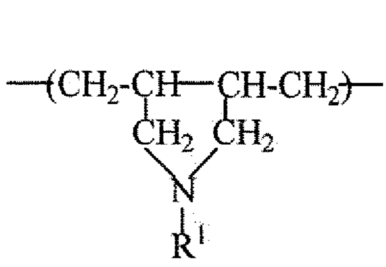

(I-a)

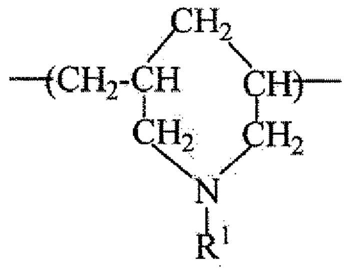

(I-b)

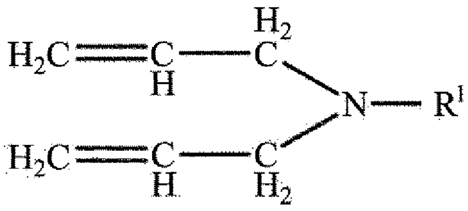

(I-c)

wherein $R^1$ represents a hydrogen atom, an alkyl group having 1 to 10 carbon atoms that may have a hydroxyl group, a cycloalkyl group having 5 to 10 carbon atoms, or an aralkyl group having 7 to 10 carbon atoms,
a constituent unit (B) having a structure represented by General Formula (I-d), General Formula (I-e), or General Formula (I-f):

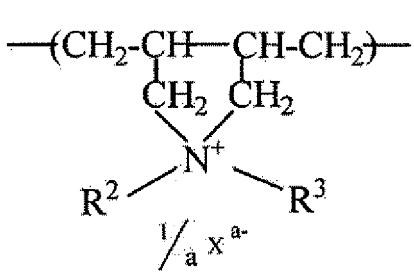

(I-d)

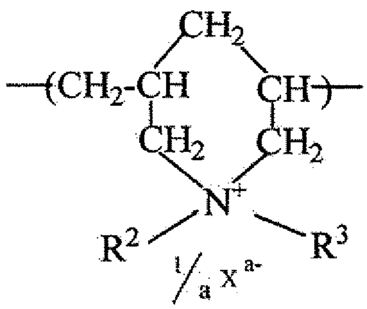

(I-e)

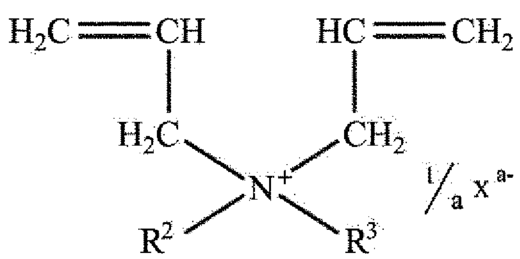

(I-f)

wherein $R^2$ and $R^3$ each independently represents a hydrogen atom, an alkyl group having 1 to 20 carbon atoms that may have a hydroxyl group, a cycloalkyl group having 5 to 20 carbon atoms, or an aralkyl group having 7 to 20 carbon atoms, $X^{a-}$ represents a counter ion, and a represents the valency number of the counter ion, and a constituent unit (C) having a structure represented by General Formula (I-g) or General Formula (1-h), or a

structure of an acid addition salt thereof:

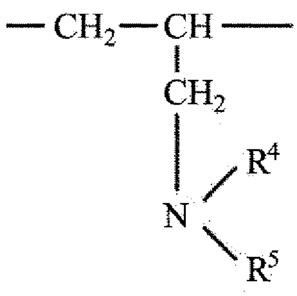

(I-g)

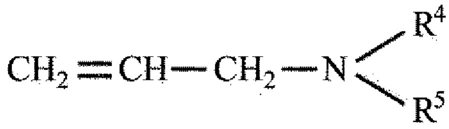

(I-h)

wherein $R^4$ and $R^5$ each independently represent a hydrogen atom, an alkyl group having 1 to 12 carbon atoms that may have a hydroxyl group, an aralkyl group having 7 to 10 carbon atoms, or a cycloalkyl group having 5 to 6 carbon atoms, and
is a compound in which a chemical structure parameter P calculated by the following Formula (1) and a molecular weight M are in a relationship satisfying the following Formula (2):

$$P = \frac{\sum_{i=1}^{n} m_i\left(\sum_{j=1}^{4} j \times a_{ij} + o_i + 2 \times s_i\right)}{\sum_{i=1}^{n} m_i} \quad \cdots (1)$$

wherein n represents the number of constituent units constituting the (di)allylamine-based compound, $m_i$ represents a molar ratio of a constituent unit i, $a_{ij}$ represents the number of j-class amines (or ammonium salts) in the constituent unit i, $o_i$ represents the number of carbonyl groups in the constituent unit i, and s; represents the number of sulfonyl groups in the constituent unit i

$$0.84 \leqq P^3/M^{1/4} \leqq 12.67 \quad \cdots (2)$$

.

2. The agent for use in raising a melting temperature (Tm value) of a nucleic acid according to claim 1, comprising a (di)allylamine-based compound in which the P and the M are in a relationship satisfying the following Formula (3):

$$2.37 \leqq P^3/M^{1/4} \leqq 6.92 \quad \cdots (3)$$

.

3. The agent for use in raising a melting temperature (Tm value) of a nucleic acid according to claim 1, comprising a (di)allylamine-based compound in which the P and the M are in a relationship satisfying the following Formula (4):

$$2.60 \leqq P^3/M^{1/4} \leqq 3.00 \quad \cdots (4)$$

.

4. The agent for use in raising a melting temperature (Tm value) of a nucleic acid according to any one of claims 1 to 3, wherein the (di)allylamine-based compound is a (di)allylamine-based (co)polymer.

5. The agent for use in raising a melting temperature (Tm value) of a nucleic acid according to any one of claims 1 to 4, wherein the nucleic acid is DNA.

6. A method for stabilizing a nucleic acid, comprising:
adding the agent for raising a melting temperature (Tm value) of a nucleic acid according to any one of claims 1 to 5 to an aqueous solution containing a nucleic acid.

7. An aqueous solution containing the agent for raising a melting temperature (Tm value) of a nucleic acid according to any one of claims 1 to 5 together with a nucleic acid.

## INTERNATIONAL SEARCH REPORT

International application No. **PCT/JP2022/044333**

**A. CLASSIFICATION OF SUBJECT MATTER**

***C12Q 1/6827***(2018.01)i; *C12N 15/11*(2006.01)n
FI: C12Q1/6827 Z ZNA; C12N15/11 Z

According to International Patent Classification (IPC) or to both national classification and IPC

**B. FIELDS SEARCHED**

Minimum documentation searched (classification system followed by classification symbols)

C12Q1/6827; C12N15/11

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br><br><br><br>Y | YAMAYOSHI, Asako et al. Selective and Robust Stabilization of Triplex DNA Structures Using Cationic Comb-type Copolymers. The Journal of Physical Chemistry B. 2017, vol. 121, pages 4015-4022, doi: 10.1021/acs.jpcb.7b01926<br>abstract, fig. 1, table 1 | 1-7<br><br><br><br>1-7 |
| Y | WO 2003/018841 A1 (THE CIR. FOR THE PROMOTION OF SCIENCE AND ENGINEERING) 06 March 2003 (2003-03-06)<br>claims, page 5, "PAA-g-Dex", example 2 | 1-7 |
| A | JP 2008-278779 A (ASAHI KASEI CORP.) 20 November 2008 (2008-11-20)<br>claims | 1-7 |
| P, X | TACHIBANA, Ami et al. Cationic copolymers that enhance wild-type-specific suppression in BNA-clamp PCR and preferentially increase the Tm of fully matched complementary DNA and BNA strands. Biology Methods and Protocols. 2022, vol. 7, no. 1, bpac009, doi: 10.1093/biomethods/bpac009<br>entire text | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 February 2023** | **21 February 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/044333**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "The form of Annex C/ST.25 text file" above shall read as "the form of ST.26.".

Form PCT/ISA/210 (continuation of first sheet) (January 2015)

**INTERNATIONAL SEARCH REPORT**
**Information on patent family members**

International application No.
**PCT/JP2022/044333**

| Patent document cited in search report | Publication date (day/month/year) | Patent family member(s) | Publication date (day/month/year) |
|---|---|---|---|
| WO 2003/018841 A1 | 06 March 2003 | US 2004/0265823 A1<br>claims, page 2, rigth column, "PAA-g-Dex", example 2<br>JP 4178194 B2<br>EP 1428890 A1<br>CN 1547616 A | |
| JP 2008-278779 A | 20 November 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015212273 A
- JP 2005058107 A
- WO 03018841 A1
- JP 2001078769 A
- JP 2008278779 A


### Non-patent literature cited in the description


- **DIEFFENBACH et al.** General concepts for PCR primer design. *PCR Methods Appl.,* 1993, vol. 3 (3), 30-37